# EUROPEAN PATENT SPECIFICATION

(11) **EP 2 856 152 B1**
(45) Date of publication and mention of the grant of the patent: **04.08.2021**
(21) Application number: 13729629.9
(22) Date of filing: 04.06.2013
(51) Int. Cl.: G01N 33/49, A61P 37/06

(54) **NEW BIOMARKERS TO ESTIMATE THE RISK OF ALLOGRAFT FAILURE AND PATIENT MORTALITY AFTER ORGAN TRANSPLANTATION**
NEUE BIOMARKER ZUM ABSCHÄTZEN DES RISIKOS VON ALLOGRAFTVERSAGEN UND PATIENTENSTERBLICHKEIT NACH ORGANTRANSPLANTATION
NOUVEAUX BIOMARQUEURS POUR ESTIMER LE RISQUE DE DEFAILLANCE D'ALLOGREFFE ET DE MORTALITE DE PATIENT A LA SUITE D'UNE TRANSPLANTATION D'ORGANE

(30) Priority: 04.06.2012 EP 12170756
(43) Date of publication of application: 08.04.2015
(73) Proprietor: NattoPharma ASA, 0283 Oslo (NO)
(72) Inventor: VERMEER, Cornelis, 6229 EV Maastrich (NL); DE BORST, Martin, 9741 CH Groningen (NL)
(74) Representative: Acapo AS
(86) International application number: PCT/EP2013/061519
(87) International publication number: WO 2013/182579

(56) References cited:
- WO-A2-2004/108748
- WO-A2-2005/054503
- US-A- 5 635 478
- KIJI T ET AL: "Relationship between immunological rejection and matrix GLA protein in cryopreserved vascular allografts", TRANSPLANTATION PROCEEDINGS, ELSEVIER INC, ORLANDO, FL; US, vol. 36, no. 8, 1 October 2004 (2004-10-01), pages 2415-2417, XP004653016, ISSN: 0041-1345, DOI: 10.1016/J.TRANSPROCEED.2004.06.032
- None

## Description

### Field of the Invention

The present invention is in the field of clinical diagnostics. In particular, the invention relates to the use of gamma-carboxyglutamate-containing proteins as biomarkers for estimating the risk of allograft failure and mortality in patients who underwent organ transplantation.

### Background of the invention

Vitamin K may occur in two different forms: K1 and K2. Whereas K1 comprises one single chemical structure (phylloquinone), K2 is a group name for the family of menaquinones (abbreviated as MK-n), which have in common a methylated naphthoquinone ring structure as the functional group, but which vary in the length of their polyisoprenoid side chain. In the generally adopted nomenclature, *n* stands for the number of isoprenyl residues in MK-n. The number of isoprenyl residues in the side chain may vary from 1 (in MK-1) to 13 (in MK-13). The different forms of vitamin K share the function as coenzyme for the posttranslational enzyme gammaglutamate carboxylase (GCCX), but substantial differences have been reported with respect to absorption, transport, and pharmacokinetics {Schurgers LJ, Vermeer C. Biochim Biophys Acta 1570 (2002) 27-32}. Whereas K1 is preferentially utilized by the liver, K2 vitamins (mainly the long-chain menaquinones MK-7 through MK-10) are readily transported to extra-hepatic tissues, such as bone, arteries and adipose tissue. Commercially available K-vitamins include K1, MK-4 and MK-7.

The product of vitamin K action is the unusual amino acid gammacarboxy-glutamic acid, abbreviated as Gla. Presently, 17 Gla-containing proteins have been discovered and in those cases in which their functions are known they play key roles in regulating important physiological processes, including haemostasis, calcium metabolism, and cell growth and survival {Berkner KL, Runge KW. J Thromb Haemostas 2 (2004) 2118-2132*}.* Since new Gla-proteins are discovered almost every second year {Viegas CS et al. Am J Pathol 175 (2009) 2288-2298*},* it is to be expected that more Gla-protein-controlled processes will be identified in the near future. In all Gla-proteins the function of which is known, the Gla-residues are essential for the activity and functionality of these proteins, whereas proteins lacking these residues are defective {Berkner KL, Runge KW. J Thromb Haemostas 2 (2004) 2118-2132*}.* The specificity with which Gla-domain structures facilitate interaction of vitamin K-dependent coagulation proteins with cell membranes is now becoming understood {Huang M et al. Nature Struct Biol 10 (2003) 751-756}. Likewise, it is well accepted that the Gla-residues of osteocalcin confer binding of the protein to the hydroxyapatite matrix of bone in a manner strongly suggestive of selectivity and functionality {Hoang QQ. Nature 425 (2003) 977-980}.

The Gla-proteins involved in haemostasis are all synthesized in the liver: four blood coagulation factors (II, VII, IX, and X) and three coagulation inhibiting proteins (C, S, and Z). These proteins are generally designated as the "hepatic Gla-proteins" referring to their origin. In the normal healthy population, vitamin K intake is sufficient to cover the requirements of the liver, so in healthy adults all coagulation factors are fully carboxylated.

Besides the hepatic Gla-proteins, we also know the extra-hepatic Gla-proteins, i.e. those Gla-proteins that are synthesized outside the liver. As will be detailed below, most extra-hepatic Gla-proteins are substantially under-carboxylated with 20-30% of the total antigen being present in the Gla-deficient (and hence inactive) state. Examples are the bone Gla-protein osteocalcin (OC) and the vascular Matrix Gla-Protein (MGP) {Knapen MH et al. Ann Int Med 111 (1989) 1001-1005*;* Cranenburg EC et al. Thromb Haemostas 104 (2010) 811-822*}.* Whereas the function of MGP as an inhibitor of soft tissue calcification is well understood {Schurgers LJ et al. Thromb Haem 100 (2008) 593-603}, the function of OC has remained a matter of debate even 30 years after its discovery. The main function of the recently discovered Gla-rich protein (GRP) is probably also related to inhibiting calcification, notably in cartilage {Cancella ML et al. Adv Nutr 3 (2012) 174-181}.

Both carboxylated (cMGP) and uncarboxylated MGP (ucMGP) species have been detected in normal human plasma {Cranenburg EC et al. Thromb. Haemostas. 104 (2010) 811-822*;* Schurgers LJ et al. Blood 109 (2007) 3279-3283}. Whereas the total fraction of ucMGP (t-ucMGP) was found to be a "disease marker" with the diagnostic potential of monitoring the amount of prevalent arterial calcium {Cranenburg EC et al. Thromb. Haemostas. 101 (2009) 359-366*},* the fraction known as desphospho-uncarboxylated MGP (dp-ucMGP) was shown to indicate overall vitamin K status and to be a "risk marker" directly associated with future calcification risk and cardiovascular mortality {Schurgers LJ et al. Clin. J. Am. Soc. Nephrol. 5 (2010) 568-575}.

Besides conformation-specific assays for dp-ucMGP, also conformation-specific assays for OC are commercially available (carboxylated OC (cOC) and uncarboxylated OC (ucOC), respectively). Either ucOC or the ratio ucOC/cOC or the ratio ucOC/total OC are often used to link vitamin K status with low bone mineral density and fracture risk {Szulc PJ et al. J. Clin. Invest. 91 (1993) 1769-1774*;* Luukinen H et al. J. Bone Miner. Res. 15 (2000) 2473-2478*,* van Summeren et al. Brit J Nutr 100 (2008) 852-858}. In general, uncarboxylated Gla-proteins are markers for vitamin K-insufficiency and for enhanced disease risk, notably of age-related diseases {McCann JC and Ames BN. Am J Clin Nutr 90 (2009) 889-907}.

Conformation-specific assays for MGP have been described by Vermeer C, inter alia in the patent literature (EP 1190259, EP 1591791, US 6,746,847, US 7,700,296, and US 8,003,075). Their diagnostic use for cardiovascular disease and rheumatoid arthritis has been demonstrated. However, these patent publications are silent about the use of uncarboxylated MGP (notably dp-ucMGP) as a marker for immune response and organ transplant failure.

Recently, dietary vitamin K intake has been associated with inflammation. High vitamin K1 intake and high circulating vitamin K1 levels were found to be correlated with low levels of the inflammation marker CRP {Shea MK et al. Am J Epidemiol 167 (2008) 313-320*}.* The authors indicate that the mechanism may not be based on the classical function of vitamin K (posttranslational protein carboxylation) but on increased expression of inflammation-related genes. Inflammation may be the result of both the genetic makeup and an acquired immune response, and is typically accompanied by increased production of cytokines, including TNF-alpha, interleukin-1 and interleukin-6.

In rejection following organ transplantation, however, a prominent role has been attributed to different mechanisms, mainly those associated with an increase of HLA-proteins and T-lymphocytes {Sun HJ et al. Transpl. Immunol. 25 (2011) 42-48*;* Loupy A et al. Nat. Rev. Nephrol. 8 (2012) 348-357}*.* A role of vitamin K or the importance of vitamin K sufficiency in this process have neither been described nor suggested so far.

The only disclosure of the use of vitamin K in transplantation patients is to treat vitamin K-deficiency-related haemorrhages characterized by a prolonged prothrombin or partial thromboplastin time during the first postoperative week to avoid hemorrhagic complications (bleeding) {Prasad GV et al. Am J Kidney Dis 33 (1999) 963-965}-Obviously, the only purpose of this *short-term* application of vitamin K is to restore the hepatic vitamin K stores which may have been exhausted peri-operatively; in this way the blood coagulation system is normalized so that postoperative bleeding is prevented.

WO 2005/054503 discloses a method for monitoring transplant rejection in a subject comprising monitoring the level of mRNA or corresponding protein expression of a number of gene transcripts, amongst which MGP mRNA and its translation product MGP, and comparing the levels prior to and at a predetermined time after transplantation. The method is silent, however, on posttranslational modification and activation of the marker proteins. Also, there is no disclosure on other Gla-proteins including osteocalcin and GRP.

Kiji et al., Transplantation Proceedings 36 (2004) 2415-2417 discloses that at 9 days after transplantation MGP expression levels (as measured on mRNA level) in transplanted rat aortas were significantly lower in allografts than in isografts. It was concluded that immunological rejection is likely to inhibit MGP expression in vascular allografts, resulting in late-onset calcification. However, only mRNA expression at tissue level was measured. There is neither a disclosure of protein synthesis and posttranslational modification nor of the use of circulating MGP fractions as a marker for allograft rejection risk.

Presently, in kidney transplant patients allograft failure is most frequently taking place between 1 and 10 years after transplantation, and it is difficult to assess who is at risk for early allograft loss and death.

There is, therefore, a need for a simple blood test that is useful to identify patients at high risk of allograft failure and mortality, which will enable clinicians to intervene and adapt medication and thus will contribute to improved treatment of patients after organ transplantation.

### Summary of the Invention

The present invention provides in one aspect a method for estimating the risk of allograft failure and patient mortality associated with allograft failure in a patient following organ transplantation which comprises the following steps:
a) assessing the vitamin K status in blood, plasma or serum drawn or taken from said patient after transplantation by measuring the degree of carboxylation of circulating extrahepatic Gla-proteins,
b) providing a reference scheme showing the vitamin K status of a reference population of healthy people wherein the degree of carboxylation of circulating extrahepatic Gla-proteins was measured in the same way as in step a)
c) comparing the value obtained in step a) with the reference scheme of step b), wherein a value outside the normal range of said reference scheme is indicative for a risk of allograft failure and/or patient mortality.

Preferably, the Gla-protein is selected from the group consisting of matrix-Gla Protein (MGP), which is the preferred embodiment, prothrombin, any one of the clotting factors VII, IX and X, coagulation-inhibiting proteins C, S and Z, osteocalcin, matrix-Gla-protein, GRP, Gas6, periostin, periostin-like factor, and any one of the four transmembrane Gla-proteins.

The degree of carboxylation of circulating extrahepatic Gla-proteins is preferably assessed from one of total uncarboxylated MGP (t-ucMGP), phospho-uncarboxylated MGP (p-ucMGP), desphospho-uncarboxylated MGP (dp-ucMGP) which is the most preferred embodiment, ucOC (also preferred), and ucGRP.

When dp-ucMGP is measured, a value below the normal range of the reference scheme is indicative for a low risk and a dp-ucMGP value greater than the normal range is indicative for a higher risk, the extent of which correlates with the deviation from the normal range.

The organ transplantation to which the present invention pertains is primarily, but not exclusively, kidney transplantation and therefore the patients are primarily kidney transplant patients.

In another aspect the present invention provides the use of a conformation-specific assay for either cOC, dp-cMGP or cGRP in the follow-up of patients after organ transplantation.

In a further aspect the present invention provides a method for estimating the risk of allograft failure and patient mortality associated with such allograft failure after organ transplantation which comprises assessing circulating uncarboxylated and/or carboxylated forms of Gla-proteins as a biomarker in the blood of said patient.

Preferably, a conformation-specific assay is used for assessing one or more circulating uncarboxylated and/or carboxylated forms of Gla-proteins in the blood or plasma of said patients. Said one or more uncarboxylated and/or carboxylated forms of Gla-proteins are preferably selected from the group consisting of uncarboxylated and/or carboxylated species of osteocalcin (OC), uncarboxylated and/or carboxylated species of matrix Gla protein (MGP), and uncarboxylated and/or carboxylated species of Gla-rich protein (GRP).

Preferably, the circulating uncarboxylated forms of Gla-proteins are assessed, i.e. dp-ucMGP, ucOC and ucGRP, as this method is basically the more sensitive compared to tests for the corresponding carboxylated species.

In another aspect the present invention provides the use of a conformation-specific assay for either cOC, dp-cMGP or cGRP in the follow-up of patients after organ transplantation.

These and other aspects of the present invention will be more fully outlined in the detailed description which follows.

### Brief Description of the Drawings

- Figure 1:: Poor vitamin K status in patients after kidney transplantation. Error bars represent SD. The difference between patients with normal and low vitamin K intake was significant at p<0.05, the difference between the healthy reference group and patients with normal vitamin K intake was significant at p<0.005 (Student t-test).
- Figure 2:: Higher plasma dp-ucMGP and ucOC are associated with increased risk of mortality. The three sub-groups represent tertiles (equal size) of the total patient population.
- Figure 3:: Higher plasma dp-ucMGP and ucOC are associated with increased risk of cardiovascular mortality.
- Figure 4:: Higher plasma dp-ucMGP and ucOC are associated with increased risk of (non-censored) graft failure.
- Figure 5:: Higher plasma dp-ucMGP and ucOC are associated with an increased risk of the composite endpoint of mortality and graft failure.
- Figure 6:: Significant fractions of both MGP and osteocalcin occur in their uncarboxylated (= vitamin K-deficient) conformation in healthy adults.
- Figure 7:: Correlation between ucOC and dp-ucMGP.

### Definitions:

The term "allograft failure", as used herein, is defined as acute or chronic loss of function of the transplanted organ and is considered an irreversible state of dysfunction of the transplanted organ. In the example of kidney transplantation, allograft failure is the loss of function so severe that the patient needs to return to renal replacement therapy (i.e. hemodialysis, peritoneal dialysis or re-transplantation). Usually this is required when GFR is <10 mL/min and patient has uremic complaints (encephalopathy, pericarditis etc), hyperkalemia, severe fluid retention etc.

Vitamin K, as used herein, refer to phylloquinone (also known as vitamin K₁); and menaquinone (also known as vitamin K2). Within the group of vitamin K2, special reference is made to menaquinone-4 (MK-4) and the long-chain menaquinones (MK-7, MK-8 and MK-9), in particular menaquinone-7 (MK-7). It is generally accepted that the naphthoquinone moiety which they have in common is the functional group, so that the mechanism of action is similar for all K vitamins. Differences may be expected, however, with respect to intestinal absorption, transport, tissue distribution, and bioavailability.

As used herein, the term "vitamin K status" refers to the extent to which various Gla-proteins have been carboxylated. Poor carboxylation means that the vitamin K supply in the tissue from which the Gla-protein originates has been too low to ensure its full carboxylation.

In general a distinction is made between hepatic vitamin K status (carboxylation of coagulation factors) and extra-hepatic vitamin K status. The liver produces the vitamin K-dependent blood coagulation factors. Insufficient hepatic vitamin K status is extremely rare; therefore, the clotting factors are no sensitive markers for vitamin K status. In the present patent application a person's vitamin K status will be regarded as poor when the circulating level of the extra-hepatic Gla-protein MGP (measured as dp-ucMGP) is above the upper normal level in healthy adults. Likewise, a person's vitamin K status is also regarded as low when the circulating level of the extra-hepatic protein Gla-protein osteocalcin is above the upper normal level in healthy adults. Dp-ucMGP originates from tissues other than the liver, mainly arteries and cartilage.

Likewise, ucOC originates primarily from bone. Moreover, evidence is provided that there is a strong correlation between the circulating levels of ucOC and dp-ucMGP, suggesting that other extra-hepatic Gla-proteins may be used similarly as markers for extra-hepatic vitamin K status.

As used herein, the term "study cohort" is defined as the population (group of subjects, group of patients) in which the particular study has been performed.

Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters set forth, the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

It is noted that, as used in this specification and the appended claims, the singular forms "a," "an," and "the," include plural referents unless expressly and unequivocally limited to one referent. Thus, for example, reference to "a monomer" includes two or more monomers.

### Detailed Description of the Invention

The present invention is based on the surprising discovery that vitamin K status, as measured by the degree of carboxylation of circulating extrahepatic Gla-proteins, is inversely correlated with allograft failure and patient mortality in patients after kidney transplantation.

The invention therefore provides the use of uncarboxylated or incompletely carboxylated species of MGP as a biomarker for monitoring the risk of transplant failure and mortality in patients following organ transplantation. The monitoring may start conveniently 1-2 months after surgery, when the patients have been stabilized. Subsequently, the monitoring may be repeated regularly, for instance once or twice a year.

While the invention is exemplified with uncarboxylated Matrix Gla-Protein (MGP), at this time seventeen Gla-proteins are known and all may be used for this purpose, i.e. the assessment of the vitamin K status in a patient following organ transplantation, and are therefore within the scope of the present invention. Gla-proteins described today include: prothrombin, clotting factors VII, IX and X, coagulation-inhibiting proteins C, S and Z, osteocalcin, matrix-Gla-protein (MGP), Gla-rich protein (GRP, also known as UCMA), Gas6, periostin, periostin-like factor and four transmembrane Gla-proteins. Uncarboxylated MGP occurs in two forms: one containing phosphoserine residues and a second form in which the serine residues have not been phosphorylated. Consequently, tests may be developed for total uncarboxylated MGP (t-ucMGP), phospho-uncarboxylated MGP (p-ucMGP) and desphospho-uncarboxylated MGP (dp-ucMGP). Since the measurement of dp-ucMGP is more sensitive than t-ucMGP and p-ucMGP, the present invention is exemplified with dp-ucMGP which is a preferred embodiment. However, it will be understood that the invention is not limited to this embodiment.

The range of circulating dp-ucMGP in apparently healthy adults is between 100 and 650 pmol/L, with a tendency to the higher end of this range at increasing age. Above 650 pmol/L is regarded as clearly abnormal, and only few individuals have values > 500 pmol/L. The average for the normal adult reference population is 450 pmol/L of plasma dp-ucMGP. These values have been assessed after measurement of >1000 apparently healthy individuals aging between 25 and 75 years; 95% of all values fall within the range of 100-500 pmol/L, which consequently is designated as "the normal range".

Vitamin K status is regarded to be inadequate or insufficient when the circulating concentrations of uncarboxylated Gla-proteins exceed the upper normal value. For dp-ucMGP the upper normal value is set at 500 picomol/L (pM).

According to the invention, a reference scheme is provided showing ranges for plasma dp-ucMGP values indicating a risk scale for mortality and graft failure. Transplantation patients are subdivided into three tertiles for circulating dp-ucMGP: <833 pmol/L; 833-1306 pmol/L and >1306 pmol/L. Subjects in tertiles with increasing dp-ucMGP had increased risk of both mortality and graft failure. Consequently, the risk scale for mortality and graft failure can be set as follows:
- Low risk: plasma dp-ucMGP < 450 pmol/L
- Slightly elevated risk: plasma dp-ucMGP 451-832 pmol/L
- Substantial risk: plasma dp-ucMGP 833-1306 pmol/L
- High risk: plasma dp-ucMGP > 1306 pmol/L

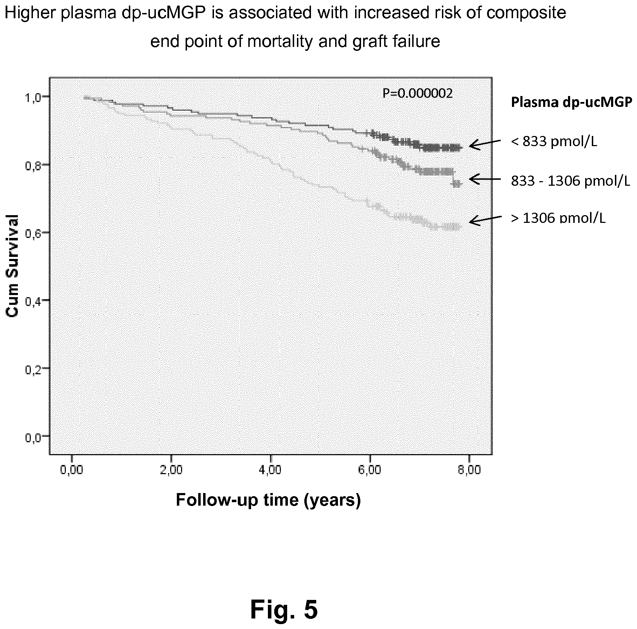

In this scheme, the lowest risk is attributed to those who have dp-ucMGP below the average value of the normal ("healthy") reference population, the three groups at increased risk are defined on the basis of the tertiles in the patient group as shown in Figure 5

Although the above scheme provides a convenient tool to classify patients, it will be clear that there is a gradual risk increase at increasing dp-ucMGP levels between 100 and 5000 pmol/L. Also, a correction factor may be used for age, gender or race. Finally, also the design of the dp-ucMGP assay and the affinity of antibodies used therein may influence the precise values given in this classification. Therefore, a package insert in the kit must contain the normal reference values and the ranges for higher risk scales whereas it remains to the medical practitioner how to interpret the patient's value against this background information.

Classification of an individual patient will take place by taking blood, usually by venipuncture, and to assess the dp-ucMGP and/or ucMGP value. The assessment is carried out according to technology which is well-known to a person skilled in the art. The observed value will be compared with the ranges given in the reference table above. In this way the patient will be classified to be: at low risk, at slightly elevated risk, at substantial risk or at high risk of organ failure and/or death. This will help the transplantation team to optimize treatment. Repeated measurement of the patient's dp-ucMGP value (e.g. every six months) will help to adequately monitor the risk profile of the patient and to implement necessary changes in treatment in an early phase.

The present invention further provides the use of other MGP species or total MGP antigen for monitoring the risk of transplant failure and mortality in transplant patients. These forms of MGP include carboxylated species (which will be higher when uncarboxylated species are lower) such as dp-cMGP, but also total uncarboxylated MGP (t-ucMGP) as a marker for arterial calcification and survival. The assessment can be performed as described, inter alia, in Cranenburg E.C.M., Koos, R., Schurgers, L.J., Magdeleyns, E., Schoonbrood, T.H.M., Landewe, R., Brandenburg, V.M., Bekers, O., Vermeer, C. Characterization and potential diagnostic value of circulating matrix Gla protein (MGP) species. Thromb. Haemostas. 104 (2010) 811-822*,* and must be compared with reference tables prepared for the corresponding MGP fraction.

In still another aspect, the present invention includes the use of conformation-specific assays for any of the other Gla-proteins, such as ucOC and cOC, for monitoring the risk of transplant failure and mortality in transplant patients. In all cases assays for uncarboxylated Gla-proteins will give increasing values at increasing vitamin K-insufficiency, whereas assays for carboxylated Gla-proteins will give decreasing values at increasing vitamin K-insufficiency. For ucOC the normal range was found to be between 0.5 and 4.0 µg/L with an upper normal value of 4.0 µg/L. These values were measured in a similar way as described for dp-ucMGP, but using a commercial kit for ucOC (GLU-OC kit from Takara, Japan).

Having now generally described the invention, the same may be more readily understood through the following reference to the experimental part including the examples and the accompanying drawings, which are provided by way of illustration and are not intended to limit the present invention unless specified. On the contrary, the invention is intended to cover all alternatives, modifications, and equivalents, which may be included within the invention as defined by the appended claims.

*Study cohort:* The study cohort in which the present invention was made has been described in detail elsewhere {de Vries AP et al. Am. J Transpl. 4 (2004) 1675-1683}. In short, the Institutional Review Board of the Groningen University approved the study protocol (METC 2001/039), which was incorporated in the outpatient follow-up of the Groningen Renal Transplant Program. The outpatient follow-up constitutes a continuous surveillance system in which patients visit the outpatient clinic with declining frequency, in accordance with American Transplantation Society guidelines, i.e. ranging from twice a week immediately after hospital discharge to twice a year long-term after transplantation. Between August 2001 and July 2003, all adult allograft recipients who survived with a functioning allograft beyond the first year after transplantation (1 year post-transplant was considered *baseline*) were eligible to participate at their next visit to the outpatient clinic (*index date*). After the baseline visit, all participants visited the outpatient clinic at least once a year. Patients who had received a combined transplantation (i.e. kidney/pancreas or kidney/liver) were invited to participate as well. Patients with known or apparent systemic illnesses (e.g. malignancies or opportunistic infections) at index date were excluded from participation. A total of 606 out of 847 (72%) eligible renal transplant recipients signed written informed consent.

***Methods:*** Relevant donor, recipient, and transplant characteristics were extracted from the Groningen Renal Transplant Database. This database holds information of all renal transplantations that have been performed at the Groningen Renal Transplantation Center since 1968. In addition, the database contains the outcomes of outpatient visits (e.g. body weight, serum creatinine, creatinine clearance based on 24h urine collection, and proteinuria) at 1 month, 6 months, 1 year, 2 years, and each following fifth year after transplantation. Extracted from the database were donor and recipient age, gender, ethnicity, primary renal disease, type and duration of dialysis therapy, type and date of transplantation, number of previous transplants, cold and warm ischemia times, number of human leukocyte antigen (HLA) mismatches, delayed graft function (i.e. days of post-transplant oliguria), cytomegalovirus (CMV) status, type of acute rejection treatment, body weight at baseline, 24h creatinine clearance, and proteinuria at baseline. Smoking status at index date was obtained through a self-report questionnaire which had been sent to the participants via mail. Standard immunosuppression consisted of the following: Azathioprine (100 mg/d) and prednisolone from 1968 until 1989. Cyclosporin standard formulation (Sandimmune, Novartis: 10 mg/kg; trough-levels of 175-200 Ig/L in the first 3 months, 150 Ig/L between 3 and 12 months post-transplant, and 100 g/L thereafter) and prednisolone (starting with 20 mg/d, rapidly tapered to 10 mg/d) from January 1989 until February 1993. Cyclosporine microemulsion (Neoral, Novartis Pharma B.V., Arnhem, the Netherlands; 10 mg/kg; trough-levels idem) and prednisolone from March 1993 until May 1996. Mycophenolate mofetil (Cellcept, Roche, Nederland B.V., Woerden, the Netherlands; 2 g/d) was added from May 1997 to date. Current medication was extracted from the medical record. Calcineurin inhibitor nephrotoxicity was defined as the discontinuation of calcineurin inhibitor use, or the conversion of one calcineurin inhibitor to the other between baseline and index date.

Blood was drawn after an 8-12h overnight fasting period to prepare serum and citrated plasma, which were subsampled and frozen at -80 °C until use. Renal allograft function was assessed as the 24h urinary creatinine clearance (CrCI), i.e. the 24h urinary creatinine excretion divided by the serum creatinine concentration. Vitamin K status was assessed from plasma dp-ucMGP concentrations as described elsewhere {Cranenburg EC et al. Thromb. Haemostas. 104 (2010) 811-822} and from ucOC using a commercial dual antibody ELISA test kit (Takara Shuzo Co. Ltd., Otsu, Shiga, Japan). An in-house control serum pool was run on all ELISA plates. The primary end-points of this study were death-censored graft failure and change in kidney function. Death-censored graft failure was defined as return to dialysis therapy or re-transplant. Change in kidney function was defined as estimated GFR (eGFR) at baseline subtracted by eGFR at the last follow-up visit, divided by time from baseline to follow-up and expressed in millilitres per minute per 1.73 m² per year. This is preferred to a slope because kidney function decrease in chronic transplant dysfunction usually does not happen in a linear manner. Calculating a slope instead of a change in eGFR therefore will underestimate a patient's true kidney function decrease. For patients who died with a functioning graft (n=78), eGFR at follow-up was defined as kidney function at the last visit to the outpatient clinic before death, and for patients with graft failure (n=42), eGFR at follow-up was defined as the eGFR at the last visit to the outpatient clinic before starting dialysis therapy or re-transplant. There was no loss to follow-up.

Baseline plasma dp-ucMGP and ucOC levels were measured by ELISA in a cohort of stable outpatient kidney transplant recipients (n=528) at 7.8±6.4 (mean ± SD) years after transplantation. Associations between dp-ucMGP levels and the composite outcome of mortality and graft failure were analyzed by multivariate Cox regression analysis adjusting for age, gender, eGFR, proteinuria, hemoglobin, waist circumference, smoking, diabetes, total cholesterol, HDL cholesterol and systolic blood pressure. Sensitivity analyses were performed in a subgroup with eGFR >30 and <90 ml/min.

***Results:*** Median plasma dp-ucMGP levels amounted 1039 pmol/L and 91% of the population was above the upper-normal level of 500 pmol/L indicating vitamin K insufficiency. After 6.4±1.7 years of follow-up beyond baseline, 171 (28%) died or developed graft failure. Plasma dp-ucMGP levels were positively associated with an increased risk of the combined endpoint of all-cause mortality and graft failure (full model hazard ratio 1.86 per 1 SD increase [95% Cl 1.23-2.82], p=0.004). Analyses per tertile of dp-ucMGP and sensitivity analyses provided similar results.

For ucOC the median circulating levels were 7.3 ng/L and 87% of the population was above the upper-normal level of 4.0 ng/L indicating vitamin K insufficiency. Like dp-ucMGP, also ucOC levels were positively associated with an increased risk of the combined endpoint of all-cause mortality and graft failure (full model hazard ratio 1.78 per 1 SD increase [95% Cl 1.15-1.53], p=0.005). Analyses per tertile of ucOC and sensitivity analyses provided similar results.

***Conclusions:*** Poor vitamin K status as concluded from either circulating dp-ucMGP or ucOC is an independent risk factor for mortality and graft failure after kidney transplantation.

### Example 1

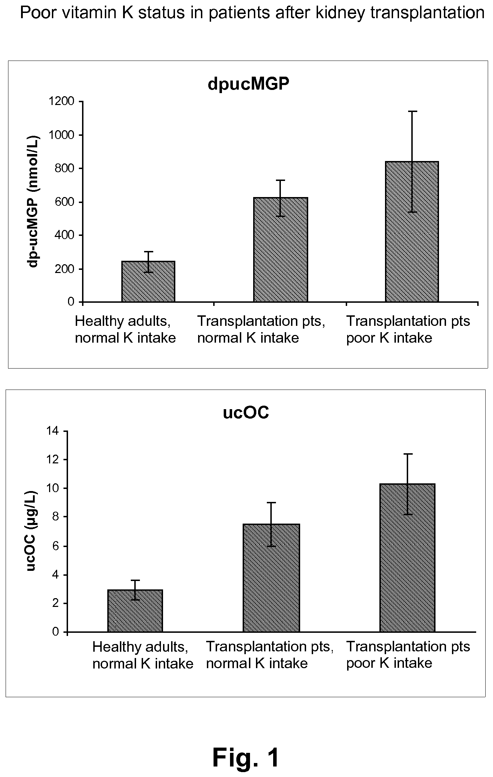

Transplantation patients have a poor vitamin K status as decided from their circulating uncarboxylated Gla-protein levels (Figure 1). In 60 kidney transplant recipients, vitamin K intake was estimated using four-day food records. Total vitamin K intake was below the recommended level (men: 120 µg/day, women: 90 µg/day) in 50% of all subjects, both in men (15/30 patients) and women (15/30 patients). Vitamin K status was assessed by measuring circulating dp-ucMGP and ucOC, and compared with 60 age- and sex-matched healthy subjects with adequate vitamin K intake. The majority of all patients had plasma dp-ucMGP and ucOC levels above the normal range, which is indicative for poor vitamin K status. Patients with vitamin K intakes below the recommended intake had higher dp-ucMGP and ucOC levels than those with adequate intakes.

### Example 2

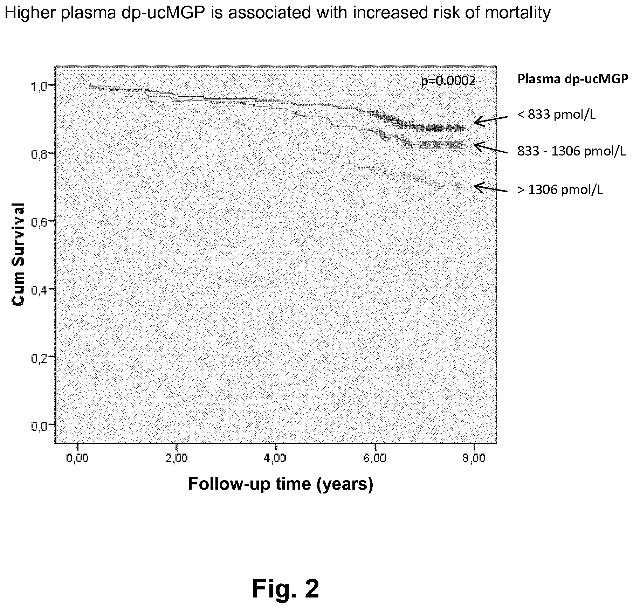

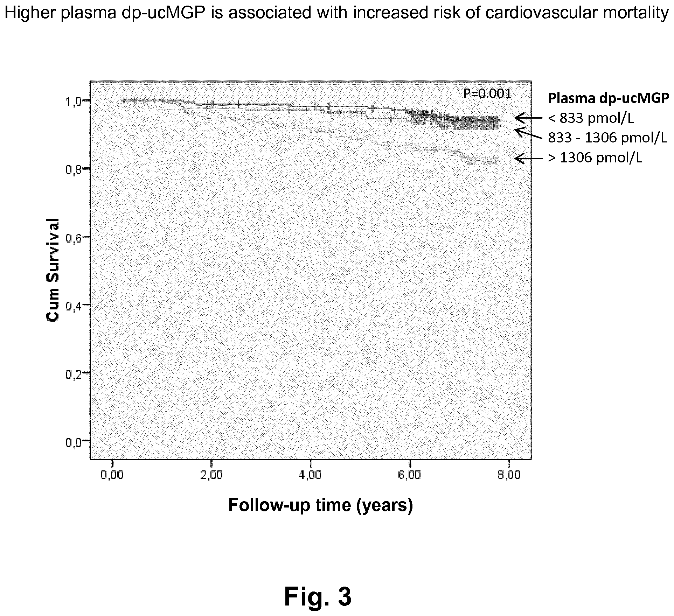

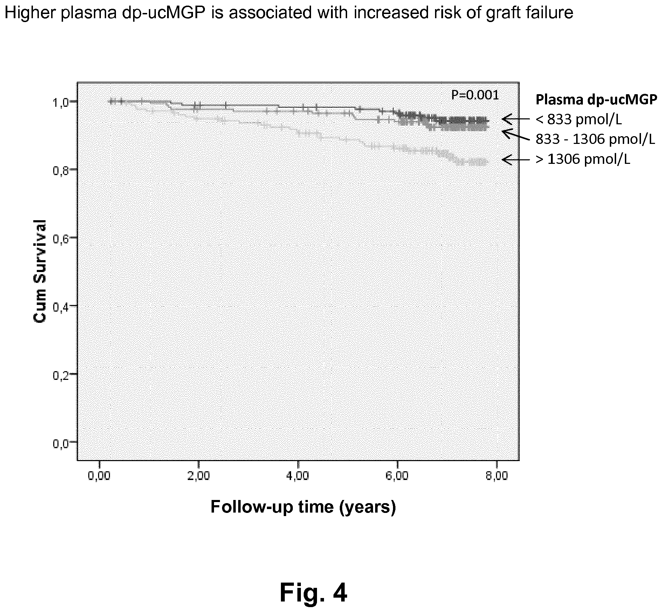

Plasma dp-ucMGP is a marker for allograft failure and transplant recipient mortality (Figures 2-5). In the study cohort, 91% of transplant patients had dp-ucMGP levels >500 pmol/L (upper level of the normal range in healthy adults). The median [IQR] in the present population (skewed) was 1039 [733-1542] pmol/L. The total study cohort (n=528) was divided into tertiles and in each tertile patients were monitored over 7 years after transplantation. In **Figures 2-5** the cumulative all-cause mortality, cardiovascular mortality, allograft failure and the composite all-cause mortality + graft failure are shown. Table 1 below shows the detailed statistics for each of these figures for dp-ucMGP. Comparable outcomes were obtained for ucOC (data not shown).

**Table 1**

| **Cox regression analysis for** **Figures 2-5** | | | |
|---|---|---|---|
| **dp-ucMGP levels (per 1 µmol/L) and outcomes** | | | |
| **Crude** | | | |
| | st. beta | 95%CI | P |
| Mortality | 1.36 | 1.19-1.55 | <0.001 |
| CV mortality | 1.43 | 1.20-1.70 | <0.001 |
| graft failure | 1.49 | 1.23-1.81 | <0.001 |
| Composite | 1.39 | 1.24-1.56 | <0.001 |

| **Age & gender adjusted** | | | |
|---|---|---|---|
| | st. beta | 95%CI | P |
| Mortality | 1.28 | 1.11-1.48 | 0.001 |
| CV mortality | 1.34 | 1.11-1.61 | 0.001 |
| graft failure | 1.56 | 1.28-1.89 | <0.001 |
| Composite | 1.33 | 1.18-1.50 | <0.001 |

| **Adjusted for age, gender, creatinine clearance, proteinuria** | | | |
|---|---|---|---|
| | st. beta | 95%CI | P |
| Mortality | 1.23 | 1.05-1.45 | 0.012 |
| CV mortality | 1.31 | 1.06-1.61 | 0.011 |
| graft failure | 1.29 | 1.01-1.65 | 0.04 |
| Composite | 1.26 | 1.09-1.44 | 0.001 |

| **Adjusted for age, gender, creatinine clearance, proteinuria, Hb, use of ACEi/ARB, waist circumference, smoking, diabetes, total cholesterol, HDL cholesterol and systolic blood pressure** | | | |
|---|---|---|---|
| | st. beta | 95%CI | P |
| Mortality | 1.20 | 1.02-1.41 | 0.02 |
| CV mortality | 1.30 | 1.07-1.60 | 0.01 |
| graft failure | 1.35 | 1.00-1.82 | 0.048 |
| Composite | 1.22 | 1.06-1.41 | 0.005 |

### Example 3

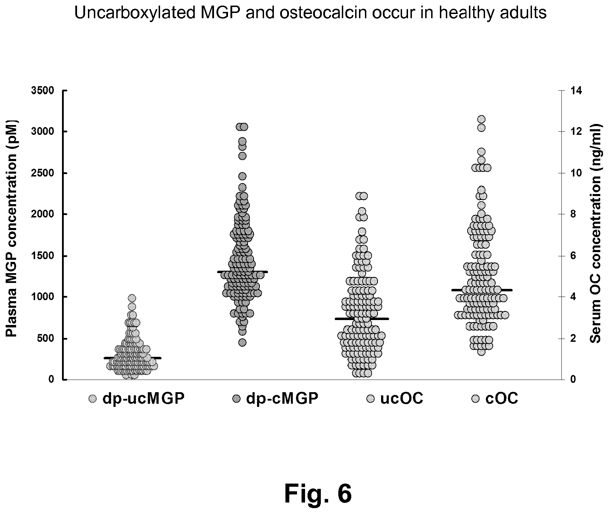

Both dp-ucMGP and ucOC are found in the circulation of the study cohort. (**Figure 6**). Both carboxylated and uncarboxylated species of osteocalcin and MGP were measured using conformation-specific assays as described elsewhere. It resulted that between 20 and 30% of the circulating antigens occurred in the uncarboxylated state. The four different tests shown in this figure were performed in samples from the same individuals.

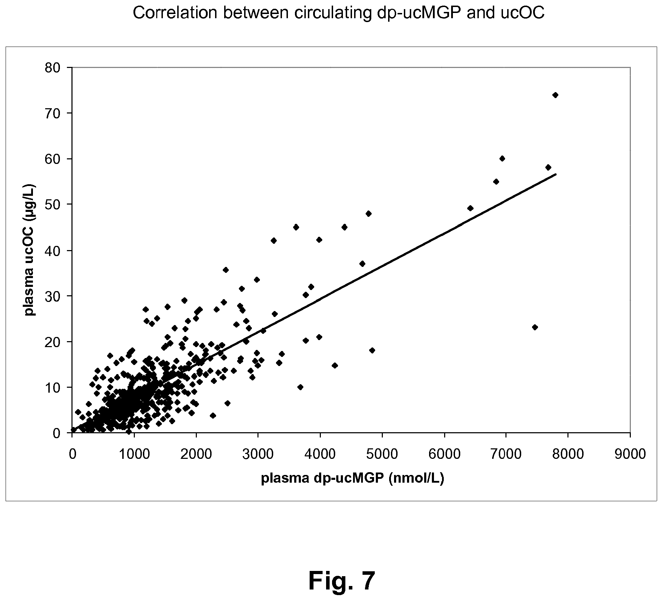

A significant correlation (R² = 0.7, p< 0.001) was found between ucOC and dp-ucMGP (**Figure 7**). This demonstrates that both dp-ucMGP and ucOC are markers that can be used to detect poor vitamin K status. A normal range for dp-ucMGP was defined between 200 and 500 pmol/L, a normal range for ucOC between 1.2 and 4.0 µg/L.

## Claims

1. A method for estimating the risk of allograft failure and patient mortality associated with allograft failure in a patient following organ transplantation which comprises
a) assessing the vitamin K status in blood, plasma or serum drawn or taken from said patient after transplantation by measuring the degree of carboxylation of circulating extrahepatic Gla-proteins,
b) providing a reference scheme showing the vitamin K status of a reference population of healthy people wherein the degree of carboxylation of circulating extrahepatic Gla-proteins was measured in the same way as in step a),
c) comparing the value obtained in step a) with the reference scheme of step b), wherein a value outside the normal range of said reference scheme is indicative for a risk of allograft failure and/or patient mortality.

2. The method of claim 1, wherein the Gla-protein is selected from the group consisting of MGP, prothrombin, any one of the clotting factors VII, IX and X, coagulation-inhibiting proteins C, S and Z, osteocalcin, matrix-Gla-protein, GRP, Gas6, periostin, periostin-like factor, and any one of the four transmembrane Gla-proteins.

3. The method of claim 2, wherein the Gla-protein is MGP.

4. The method of any one of claims 1 to 3, wherein said degree of carboxylation of circulating extrahepatic Gla-proteins is assessed from one of total uncarboxylated MGP (t-ucMGP), phospho-uncarboxylated MGP (p-ucMGP), desphospho-uncarboxylated MGP (dp-ucMGP), ucOC and ucGRP.

5. The method of any one of claims 1 to 4, wherein a dp-ucMGP value below the normal range of said reference scheme is indicative for a low risk and a dp-ucMGP value greater than the normal range is indicative for a higher risk, the extent of which correlates with the deviation from the normal range.

6. A method of any one of claims 1 to 5, wherein said organ transplantation is kidney transplantation and said patient is kidney transplantation patient.

7. The method of any one of claims 1 to 3, wherein said degree of carboxylation of circulating extrahepatic Gla-proteins is assessed from one of cOC, dp-cMGP and cGRP.

8. The method of any one of claims 1 to 7, wherein assays for uncarboxylated Gla-proteins give increasing values at increasing vitamin K-insufficiency and assays for carboxylated Gla-proteins give decreasing values at increasing vitamin K-insufficiency.

## Patentansprüche

1. Verfahren zum Abschätzen des Risikos eines Allograftversagens und einer mit Allograftversagen verbundenen Patientensterblichkeit bei einem Patienten nach einer Organtransplantation, das folgende Schritte aufweist:
a) Beurteilen des Vitamin-K-Status in Blut, Plasma oder Serum, das dem Patienten nach einer Transplantation abgenommen oder entnommen wurde, durch Messen des Grades der Carboxylierung von zirkulierenden extrahepatischen Gla-Proteinen,
b) Bereitstellen eines Referenzschemas, das den Vitamin-K-Status einer Referenzpopulation gesunder Menschen zeigt, wobei der Grad der Carboxylierung von zirkulierenden extrahepatischen Gla-Proteinen auf die gleiche Weise wie in Schritt a) gemessen wurde,
c) Vergleichen des in Schritt a) erhaltenen Wertes mit dem Referenzschema von Schritt b), wobei ein Wert außerhalb des Normalbereichs des Referenzschemas auf ein Risiko von Allograftversagen und/oder Patientensterblichkeit hinweist.

2. Verfahren nach Anspruch 1, wobei das Gla-Protein ausgewählt ist aus der Gruppe bestehend aus MGP, Prothrombin, einem der Gerinnungsfaktoren VII, IX und X, gerinnungshemmenden Proteinen C, S und Z, Osteocalcin, Matrix-Gla-Protein, GRP, Gas6, Periostin, periostinähnlichem Faktor und einem der vier transmembranen Gla-Proteine.

3. Verfahren nach Anspruch 2, wobei das Gla-Protein MGP ist.

4. Verfahren nach einem der Ansprüche 1 bis 3, wobei der Grad der Carboxylierung von zirkulierenden extrahepatischen Gla-Proteinen aus einem der Gruppe von gesamtes uncarboxyliertes MGP (t-ucMGP), phospho-uncarboxyliertes MGP (p-ucMGP), desphospho-uncarboxyliertes MGP (dp-ucMGP), ucOC und ucGRP bestimmt wird.

5. Verfahren nach einem der Ansprüche 1 bis 4, wobei ein dp-ucMGP-Wert unterhalb des Normalbereichs des Referenzschemas auf ein niedriges Risiko hinweist und ein dp-ucMGP-Wert größer als der Normalbereich auf ein höheres Risiko hinweist, dessen Ausmaß mit der Abweichung vom Normalbereich korreliert.

6. Verfahren nach einem der Ansprüche 1 bis 5, wobei die Organtransplantation eine Nierentransplantation ist und der Patient ein Nierentransplantationspatient ist.

7. Verfahren nach einem der Ansprüche 1 bis 3, wobei der Grad der Carboxylierung von zirkulierenden extrahepatischen Gla-Proteinen aus einem der Gruppe von cOC, dp-cMGP und cGRP bestimmt wird.

8. Verfahren nach einem der Ansprüche 1 bis 7, wobei Assays für nicht-carboxylierte Gla-Proteine bei steigender Vitamin-K-Insuffizienz steigende Werte ergeben und Assays für carboxylierte Gla-Proteine bei steigender Vitamin-K-Insuffizienz abnehmende Werte ergeben.

## Revendications

1. Procédé pour l'estimation du risque de défaillance d'allogreffe et de mortalité de patient associée à une défaillance d'allogreffe chez un patient à la suite d'une transplantation d'organe qui comprend:
a) l'évaluation de l'état de la vitamine K dans du sang, du plasma ou du sérum soutiré ou prélevé à partir dudit patient après la transplantation en mesurant le degré de carboxylation de protéines Gla extra-hépatiques qui circulent,
b) la fourniture d'un schéma de référence montrant l'état de la vitamine K d'une population de référence de personnes en bonne santé où le degré de carboxylation de protéines Gla extra-hépatiques qui circulent a été mesuré de la même façon que dans l'étape a),
c) la comparaison de la valeur obtenue dans l'étape a) avec le schéma de référence de l'étape b), où une valeur en dehors du domaine normal dudit schéma de référence est représentative d'un risque de défaillance d'allogreffe et/ou de mortalité de patient.

2. Procédé selon la revendication 1, dans lequel la protéine Gla est choisie dans le groupe constitué de : MGP, prothrombine, un quelconque des facteurs de coagulation VII, IX et X, protéines inhibant la coagulation C, S et Z, ostéocalcine, protéine matrice-Gla, GRP, Gas6, périostine, facteur de type périostine et une quelconque des quatre protéines Gla transmembranaires.

3. Procédé selon la revendication 2, dans lequel la protéine Gla est MGP.

4. Procédé selon l'une quelconque des revendications 1 à 3, dans lequel ledit degré de carboxylation de protéines Gla extra-hépatiques qui circulent est évalué à partir d'une protéine parmi MGP non carboxylée totale (t-ucMGP), MGP phospho-non carboxylée (p-ucMGP), MGP déphospho-non carboxylée (dp-ucMGP), ucOC et ucGRP.

5. Procédé selon l'une quelconque des revendications 1 à 4, dans lequel une valeur de dp-ucMGP en dessous du domaine normal dudit schéma de référence est représentative d'un risque faible et une valeur de dp-ucMGP supérieure au domaine normal est représentative d'un risque plus fort, dont l'ampleur est en corrélation avec l'écart à partir du domaine normal.

6. Procédé selon l'une quelconque des revendications 1 à 5, dans lequel ladite transplantation d'organe est une transplantation de rein et ledit patient est un patient avec une transplantation de rein.

7. Procédé selon l'une quelconque des revendications 1 à 3, dans lequel ledit degré de carboxylation de protéines Gla extra-hépatiques qui circulent est évalué à partir d'une parmi cOC, dp-cMGP et cGRP.

8. Procédé selon l'une quelconque des revendications 1 à 7, dans lequel les analyses pour des protéines Gla non carboxylées donnent des valeurs croissantes à une insuffisance de vitamine K croissante et les analyses pour des protéines Gla carboxylées donnent des valeurs décroissantes à une insuffisance de vitamine K croissante.
